# FASCICULE DE BREVET EUROPEEN

(11) **EP 2 079 377 B1**
(45) Date de publication et mention de la délivrance du brevet: **01.07.2015**
(21) Numéro de dépôt: 07800668.1
(22) Date de dépôt: 01.10.2007
(51) Int. Cl.: A61B 17/32, H02P 25/02, A61B 17/16, A61B 17/00

(54) **PROCEDE DE COMMANDE BIDIRECTIONNELLE D'UN MOTEUR ELECTRIQUE POUR INSTRUMENT A USAGE CHIRURGICAL OU DENTAIRE**
VERFAHREN ZUR BIDIREKTIONALEN STEUERUNG EINES ELEKTROMOTORS FÜR EIN CHIRURGISCHES ODER DENTALES INSTRUMENT
BIDIRECTIONAL CONTROL METHOD FOR AN ELECTRIC MOTOR FOR SURGICAL OR DENTAL INSTRUMENT

(30) Priorité: 05.10.2006 EP 06020905
(43) Date de publication de la demande: 22.07.2009
(73) Titulaire: Bien-Air Holding SA, 2500 Bienne 6 (CH)
(72) Inventeur: GALLINA, Marco, 3232 Tschugg (CH)
(74) Mandataire: Ravenel, Thierry Gérard Louis
(86) Numéro de dépôt international: PCT/CH2007/000482
(87) Numéro de publication internationale: WO 2008/040135

(56) Documents cités:
- US-A- 5 235 261
- US-A- 6 013 991
- US-A- 6 025 683

## Description

La présente invention concerne un procédé de commande bidirectionnelle d'un moteur électrique pour instrument à usage chirurgical ou dentaire. Plus précisément, la présente invention concerne un tel procédé qui permet de réduire sensiblement les à-coups transmis par le manche de l'instrument à la main du praticien lors du changement du sens de rotation du moteur électrique.

Que ce soit dans le domaine chirurgical ou dentaire, il est connu d'utiliser des instruments à main pour découper les tissus durs et mous, y compris les cartilages et les ligaments d'un patient. Dans le domaine particulier de l'oto-rhino-laryngologie, ces instruments à main sont notamment utilisés pour libérer les sinus d'un patient. Mais de tels instruments s'utilisent également lors d'examens arthroscopiques ou bien encore dans le domaine dentaire. De tels instruments à main sont bien connus dans le domaine de la technique concerné sous leur dénomination anglo-saxonne de "shaver" ou de "microdebrider".

Des instruments à main du genre mentionné ci-dessus comprennent essentiellement une lame coupante qui est entraînée alternativement dans un sens et dans l'autre par un moteur électrique logé dans le manche de l'instrument. Le mouvement rotatif de fa lame coupante selon l'axe de symétrie longitudinale du manche de l'instrument permet une meilleure découpe des tissus et une évacuation plus aisée des déchets.

Le mouvement oscillant de la lame coupante est classiquement obtenu au moyen d'un moteur électrique relié cinématiquement à la lame et que l'on fait tourner alternativement dans un sens et dans le sens opposé. Dans les instruments à main actuellement disponibles sur le marché, la fréquence d'oscillation de la lame coupante est typiquement de 1 à 5 Hz, ce qui signifie que la lame effectue de un à cinq cycles complets ou allers-retours par seconde. Durant un cycle, la lame effectue classiquement de 2 à 6 tours dans un sens, et le même nombre de tours dans le sens opposé.

Si la fréquence d'oscillation retenue pour la lame coupante est de 3 Hz, cela signifie que la durée de la moitié d'un cycle, autrement dit la durée d'un aller simple de cette lame, est approximativement de 170 ms. Dans les systèmes actuels, on accélère continûment le moteur depuis l'arrêt jusqu'à une vitesse maximum, puis on lui impose une rampe de décélération en prévision de l'inversion de son sens de rotation. A titre indicatif, on accélère le moteur pendant environ les trois quarts de la durée de l'aller simple, soit pendant sensiblement 126 ms, et on réserve les 44 ms restantes pour la décélération du moteur et son arrêt complet avant le changement de sens de rotation.

En effet, la consigne de commande appliquée aux bornes du moteur est classiquement de forme triangulaire. Or, le courant dans le moteur étant proportionnel à la pente de la tension appliquée aux bornes dudit moteur, il est habituel d'appliquer une tension avec une pente la plus faible possible, ceci afin d'éviter que ne circulent dans le moteur des courants trop intenses qui provoqueraient un échauffement dudit moteur et un vieillissement prématuré de celui-ci. C'est pourquoi on réserve la majeure partie de la durée d'un aller simple à l'accélération du moteur. Pour pouvoir, dans ce laps de temps, faire tourner la lame coupante par exemple de six tours dans un sens donné, le moteur doit atteindre une vitesse de l'ordre de 45'000 tours/minute au bout de sa rampe d'accélération, ce qui signifie que l'on ne dispose plus ensuite que d'environ 40 ms pour à nouveau ralentir le moteur et l'arrêter en vue de sa prochaine inversion de sens. On comprendra que, lancé à une telle vitesse et vu le faible laps de temps dont on dispose pour le freiner, le moteur est soumis à des chocs mécaniques violents dus à l'inertie de son rotor. Ces chocs se transmettent à la main du praticien à la même fréquence que la fréquence d'inversion du sens de rotation du moteur, autrement dit à une fréquence très élevée, ce qui oblige le praticien à maintenir fermement son instrument en main, d'où une fatigue pour le praticien et une diminution de la précision de son geste opératoire.

Dans le cas où la consigne de commande aux bornes du moteur électrique est de forme triangulaire, il pourrait être envisagé de réserver pour l'accélération du moteur un temps égal à celui utilisé pour sa décélération, ce qui, autrement dit, équivaudrait à diminuer le temps de la fraction du cycle consacré à l'accélération dudit moteur. En laissant davantage de temps au moteur pour décélérer, on réduirait peut-être légèrement la violence des à-coups dus à l'inertie du rotor du moteur, mais ce gain serait pour l'essentiel compensé par le fait qu'il faudrait accélérer plus rapidement encore le moteur pour respecter les consignes de fonctionnement de la lame. US-A-6025683 décrit un procédé de commande bidirectionnelle d'un moteur électrique qui entraîne alternativement dans un sens et dans le sens opposé une lame coupante d'un instrument chirurgical.

La présente invention a pour but de remédier aux inconvénients susmentionnés ainsi qu'à d'autres encore en procurant un procédé de commande bidirectionnelle d'un moteur du genre susmentionné qui permet notamment de réduire sensiblement les à-coups provoqués par l'inversion du sens de rotation du moteur.

A cet effet, la présente invention concerne un procédé de commande bidirectionnelle d'un moteur électrique qui entraîne alternativement dans un sens et dans le sens opposé une lame coupante d'un instrument chirurgical ou dentaire, caractérisé en ce qu'il consiste à appliquer au moteur, au cours d'un cycle entre deux inversions successives du sens d'entraînement de la lame coupante, un signal de commande qui présente un profil dont la pente, en valeur absolue, est essentiellement inférieure durant une partie centrale du cycle à la pente, toujours en valeur absolue, d'une partie initiale dudit cycle au cours de laquelle on accélère le moteur et d'une partie finale du cycle au cours de laquelle on décélère le moteur.

Grâce à ces caractéristiques, la présente invention procure un procédé de commande bidirectionnelle d'un moteur électrique pour instrument à usage chirurgical ou dentaire qui permet de réduire considérablement les chocs dus à l'inertie du rotor du moteur lorsque ce dernier est ralenti et arrêté en prévision du prochain changement de sens de rotation. En effet, comme on veut pouvoir, entre deux changements de sens successifs du moteur, respecter une plage durant laquelle ledit moteur subit une accélération, respectivement une décélération, moindres qu'au cours de la phase initiale de démarrage et de la phase finale de ralentissement et d'arrêt, cela signifie que, pour un nombre de tours et une fréquence d'oscillation donnés du moteur, on dispose de moins de temps que dans l'art antérieur pour accélérer ledit moteur. La vitesse maximum de rotation du moteur conformément au procédé selon l'invention est donc sensiblement inférieure à celle que l'on observait antérieurement, ce qui signifie que l'inertie du rotor du moteur est moindre et donc que les chocs ressentis lors de l'inversion du sens du moteur sont atténués. La présente invention procure ainsi un procédé de commande d'un moteur électrique pour un instrument chirurgical ou dentaire dont la tenue en main est beaucoup plus confortable et bien moins fatigante pour le praticien. On notera d'autre part que la précision du geste chirurgical est accrue.

Selon une caractéristique complémentaire du procédé de l'invention, on commence par accélérer le moteur, puis on le maintient à vitesse constante avant de le décélérer et de l'arrêter en prévision de sa prochaine inversion de sens.

Cette manière de commander le moteur répond à la définition générale de l'invention puisque, durant la fraction du cycle où l'on maintient le moteur à vitesse constante, la pente du signal de commande appliqué au moteur est nulle et est donc bien inférieure à la pente de ce même signal appliqué audit moteur durant les périodes d'accélération et de décélération.

Selon encore une autre caractéristique de l'invention, la forme du signal de commande appliqué au moteur est sensiblement carrée ou rectangulaire ou bien encore trapézoïdale.

Préférentiellement, le moteur tourne à une vitesse de l'ordre de 30'000 tours/minute durant son palier à vitesse constante.

Ce chiffre de 30'000 tours/minute est à rapprocher de celui de 45'000 tours/minute cité plus haut en liaison avec le procédé de commande d'un moteur électrique pour instrument chirurgical ou dentaire selon l'art antérieur. En effet, la Demanderesse a démontré que, quand bien même on réduit la vitesse maximale de rotation du moteur de l'ordre de 33%, on conserve toute l'efficacité de découpe des tissus de la lame coupante. Partant de là, la Demanderesse a su surmonter un préjugé très répandu dans le domaine technique de l'invention selon lequel plus la vitesse de rotation du moteur est élevée, meilleure est la qualité de découpe des tissus.

Selon une autre caractéristique de l'invention, si la fréquence d'oscillation de la lame coupante est de 3 Hz et si cette lame effectue six tours dans chaque sens, on accélère le moteur pendant environ 57 ms, on le maintient à vitesse constante pendant environ 83 ms et on le décélère pendant environ 30 ms avant de l'arrêter et d'inverser son sens de rotation.

Bien que les vitesses de rotation du moteur recherchées soient moindres dans le cadre de la présente invention que dans le cas de l'art antérieur, on réserve toutefois une durée plus courte pour atteindre la vitesse maximale recherchée, de sorte que, l'un dans l'autre, la pente suivie par la consigne de commande appliquée aux bornes du moteur est plus prononcée. Or, comme déjà mentionné précédemment, le courant dans le moteur est proportionnel à la consigne de commande appliquée aux bornes de celui-ci. Par conséquent, durant le bref instant au cours duquel on accélère le moteur, le pic de courant dans le moteur est plus élevé qu'il ne l'était dans l'art antérieur, sans que la Demanderesse n'ait pu néanmoins constater d'effet indésirable pour le moteur. Ici aussi, la Demanderesse a surmonté le préjugé fort tenace dans le domaine technique selon lequel il ne faut jamais soumettre un moteur ou micromoteur tel que ceux qui équipent les instruments à main chirurgicaux ou dentaires à de fortes intensités de courant au risque de les voir se détériorer rapidement.

La présente invention procure également une souplesse remarquable en ce qui concerne les paramètres de commande du moteur. En effet, on a vu précédemment dans la discussion sur l'art antérieur que pour respecter les consignes de fonctionnement de la lame coupante (oscillations à une fréquence de 3 Hz, 6 tours dans chaque sens), le moteur devait atteindre une vitesse de l'ordre de 45'000 tours/minute en environ 126 ms, les 44 ms restantes étant consacrées à la décélération du moteur en vue de son changement de sens de rotation. Or, si l'on souhaite augmenter le temps consacré à la phase de décélération du moteur, cela se fait inévitablement au détriment du temps réservé à la phase d'accélération de ce même moteur. Par conséquent, le moteur disposant de moins de temps, il devra accélérer plus vite encore pour effectuer le nombre de tours requis afin de respecter les consignes de fonctionnement de la lame coupante, de sorte que la diminution de la violence des à-coups que l'on peut escompter en laissant au moteur davantage de temps pour décélérer est pour l'essentiel compensée par l'augmentation de la vitesse d'accélération du moteur.

On notera par ailleurs qu'à une vitesse du moteur de 45'000 tours/minute correspond une tension appliquée sur les bornes dudit moteur de l'ordre de 45 Volts. Par conséquent, si l'on cherche à augmenter davantage encore la vitesse de rotation du moteur, il faudra appliquer à ses bornes une tension plus importante qui risque de dépasser les 50 Volts. Or, dans le domaine médical et dentaire, on cherche, pour des raisons bien évidentes de sécurité, à ne pas excéder les 50 Volts comme tension d'alimentation des instruments électriques utilisés. Par conséquent, dans l'état actuel de la technique, on jouit d'une très faible marge de manoeuvre pour agir sur les paramètres de commande du moteur. On peut même, le cas échéant, ne plus pouvoir respecter les consignes de fonctionnement de la lame coupante car on sera limité par les performances du moteur. Au contraire, dans le cadre de la présente invention, étant donné que le moteur monte moins haut en vitesse, on dispose d'une plus grande marge de manoeuvre en ce qui concerne la tension appliquée aux bornes de ce moteur pour adapter sa vitesse en fonction des consignes de fonctionnement de la lame coupante.

D'autre caractéristiques et avantages de la présente invention ressortiront plus clairement de la description détaillée qui suit d'un exemple de mise en oeuvre du procédé selon l'invention, cet exemple étant donné à titre purement illustratif et non limitatif seulement, en liaison avec le dessin annexé sur lequel:
- la figue 1A est une vue en perspective d'un instrument à usage chirurgical ou dentaire muni d'une lame coupante tournante;
- la figure 1B est une vue à plus grande échelle de la zone entourée d'un cercle sur la figure 1A;
- la figure 2A est une représentation graphique d'une première forme de tension appliquée aux bornes du moteur de l'instrument selon un procédé de commande de l'art antérieur;
- la figure 2B est une représentation graphique d'une seconde forme de tension appliquée aux bornes du moteur de l'instrument selon un procédé de commande de l'art antérieur, et
- la figure 3 est une représentation graphique d'une tension de commande de forme carrée ou rectangulaire appliquée aux bornes du moteur de l'instrument selon le procédé de commande de l'invention;
- la figure 4 est une représentation graphique d'une tension de commande appliquée au moteur de forme trapézoïdale, et
- la figure 5 est une représentation graphique d'une tension de commande appliquée au moteur de forme pseudo-sinusoïdale.

La présente invention procède de l'idée générale inventive qui consiste à appliquer aux bornes du moteur d'un instrument chirurgical ou dentaire muni d'une lame coupante une tension de forme trapézoïdale permettant d'accélérer le moteur, puis de le maintenir à vitesse constante avant de le décélérer et de l'arrêter en prévision de sa prochaine inversion de sens. Avec un tel procédé de commande, on réduit la vitesse maximale atteinte par le moteur entre deux changements de sens successifs, ce qui permet de réduire les chocs dus à l'inertie du rotor du moteur tout en respectant les consignes de fonctionnement de la lame coupante et en conservant inchangées les propriétés de découpe des tissus de cette lame.

Dans ce qui suit, on décrira l'invention en liaison avec un instrument chirurgical destiné notamment à l'oto-rhino-laryngologie et comprenant à cet effet une lame coupante qui tourne alternativement dans un sens et dans le sens contraire. Il va de soi que cet exemple est donné à titre purement illustratif seulement et que l'invention s'applique de manière identique à tout autre type d'instrument chirurgical ou dentaire comprenant une lame coupante tournante.

De même, dans tout ce qui suit, on considèrera que la lame coupante oscille à une fréquence de 3 Hz, autrement dit qu'elle effectue trois allers-retours par seconde, et qu'elle effectue six tours complets sur elle-même entre chaque inversion de sens. Il s'agit, bien entendu, d'un exemple purement illustratif seulement, d'autres consignes de fonctionnement (fréquence, nombre de tours) pouvant être imposées à la lame. De même, en fonction du rapport de multiplication ou de démultiplication de la chaîne cinématique reliant le moteur à la lame, la vitesse du moteur pourra être différente des vitesses mentionnées dans la présente demande.

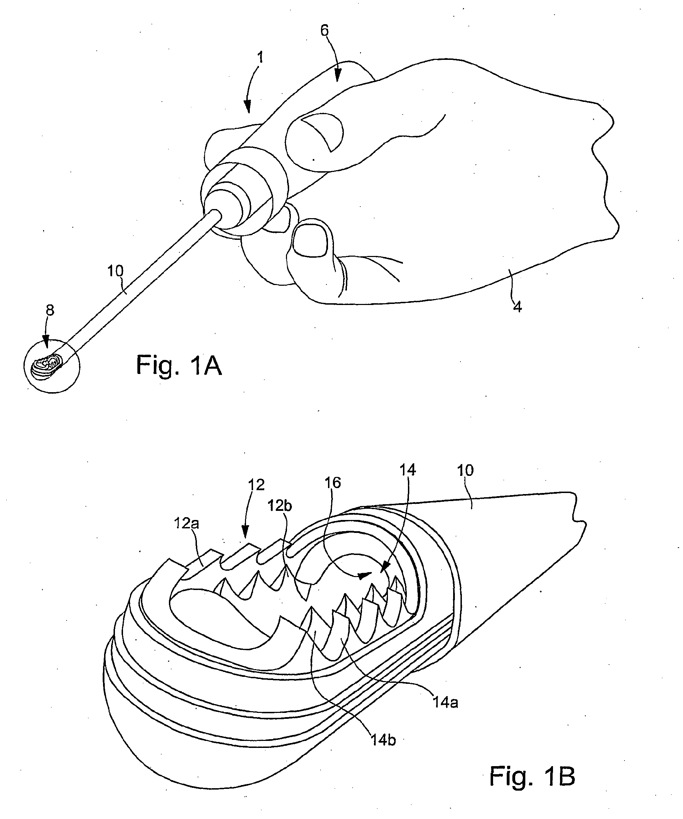

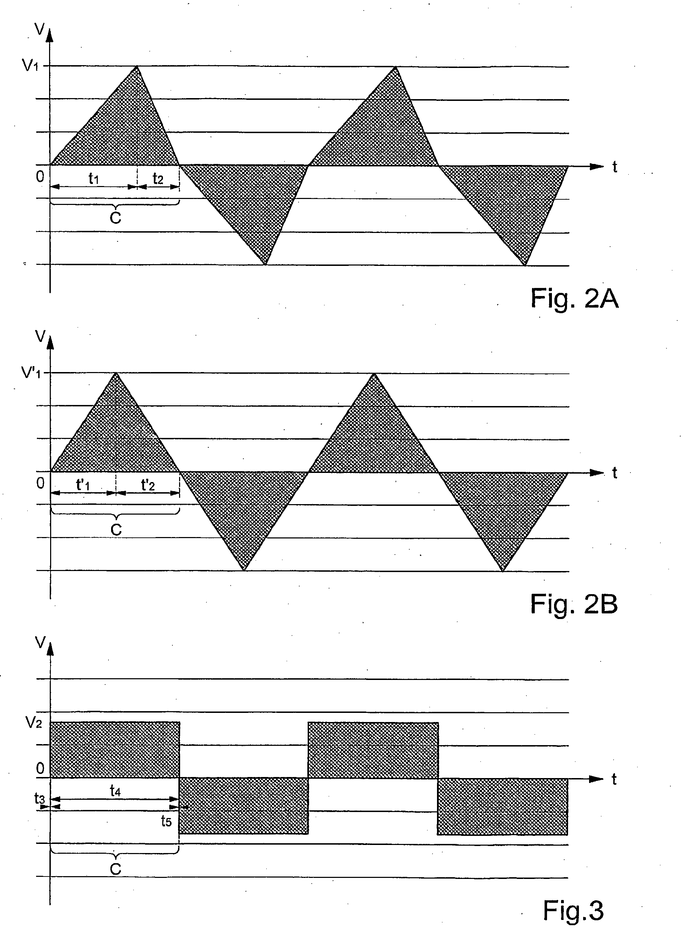

Un instrument à main chirurgical destiné à l'oto-rhino-laryngologie et plus particulièrement à la dissection de tissus qui peuvent obstruer les sinus d'un patient est représenté en perspective à la figure 1. Désigné dans son ensemble par la référence numérique générale 1, cet instrument comprend pour l'essentiel un manche 2 par lequel le praticien 4 tient l'instrument en main. Un corps 6 sensiblement cylindrique qui renferme notamment le moteur d'entraînement d'une lame coupante 8 prolonge le manche 2 à angle droit. Le corps 6 de l'instrument à main 1 est lui-même prolongé par une canule 10 à l'extrémité libre de laquelle est agencée la lame coupante 8. Comme on peut le voir à l'examen de la figure 1 et mieux encore sur la figure 2 qui est une vue à plus grande échelle de la région entourée d'un cercle sur la figure 1, la lame coupante 8 comprend deux dentures 12 et 14 disposées de manière symétrique de part et d'autre de l'axe longitudinal de la canule 10 et formées chacune d'une double rangée de dents respectivement 12a, 12b et 14a, 14b. Une telle lame coupante 8 est capable de tourner sur elle-même dans les deux sens rotation et de découper les tissus gênants. Les résidus de cette opération sont ensuite évacués par un conduit d'aspiration 16 qui s'étend à l'intérieur de la canule 10 afin d'être éliminés.

La figure 2A est une représentation graphique d'une première forme de tension appliquée aux bornes du moteur de l'instrument selon un procédé de commande de l'art antérieur. Sur ce graphe, le temps t est représenté en abscisse et la tension V appliquée aux bornes du moteur est représentée en ordonnée. On notera que la vitesse de rotation du moteur est directement proportionnelle à la tension appliquée. Dans ce qui suit, on appellera cycle C un aller simple du moteur. En tenant compte du fait que la lame coupante oscille à la fréquence de 3 Hz, la durée d'un cycle est donc approximativement de 170 ms.

Comme on peut le constater à l'examen de la figure 2A, le moteur est continûment accéléré pendant une durée t₁ d'environ 126 ms jusqu'à ce que la tension appliquée atteigne une valeur V₁ correspondant à une vitesse de rotation du moteur de l'ordre de 45'000 tours/minute. Le moteur est ensuite ralenti et immobilisé en prévision du changement de son sens de rotation sur une durée t₂ de l'ordre de 44 ms.

La vitesse de rotation à laquelle est porté le moteur est donc très élevée et le temps réservé à sa décélération très bref, de sorte que le moteur est soumis à des à-coups très intenses en raison de l'inertie de son rotor. Ces à-coups se transmettent à la main du praticien via le manche de l'instrument, rendant le travail du praticien pénible et fatigant.

Pour remédier au problème ci-dessus, il peut être envisagé d'allouer au moteur un temps plus long pour ralentir et s'arrêter avant la prochaine inversion de sens. Or, si l'on souhaite augmenter le temps consacré à la phase de décélération du moteur, cela se fait inévitablement au détriment du temps réservé à la phase d'accélération de ce même moteur. Ce cas est illustré à la figure 2B sur laquelle on voit que le temps t'₁ durant lequel le moteur est accéléré est égal au temps t'₂ durant lequel le moteur est décéléré puis arrêté avant de repartir en sens inverse, ce temps valant sensiblement 85 ms. Dans un tel cas, pour respecter les consignes de fonctionnement de la lame coupante, à savoir trois cycles ou allers-retours complets par seconde à raison de six tours dans chaque sens, il faut que le moteur accélère plus vite encore car il dispose de moins de temps pour effectuer le nombre de tours requis de sorte que la diminution de la violence des à-coups que l'on peut escompter en laissant au moteur davantage de temps pour décélérer est pour l'essentiel compensée par l'augmentation de la vitesse d'accélération du moteur.

Dans le cas idéal, la tension de commande appliquée aux bornes du moteur conformément au procédé selon l'invention présenterait une forme sensiblement carrée ou rectangulaire comme illustré à la figure 3. Autrement dit, on consacrerait un temps t₃ infiniment court de la durée d'un cycle C entre deux inversions successives du sens de rotation du moteur à l'accélération du moteur, puis on maintiendrait le moteur à vitesse constante pendant un temps t₄ correspondant à la majeure partie de la durée du cycle C avant de décélérer le moteur sur une période de temps à nouveau infiniment courte t₅. Une telle forme de signal de commande appliqué au moteur répond à la définition générale de la présente invention. En effet, la pente du signal de commande est nulle durant la partie centrale t₄ du cycle C et donc inférieure à la pente de la partie initiale t₃ dudit cycle C au cours de laquelle on accélère le moteur et d'une partie finale t₅ du cycle C au cours de laquelle on décélère le moteur. On constate d'autre part que la tension V₂ appliquée au moteur est sensiblement inférieure à la tension V₁, V'₁.

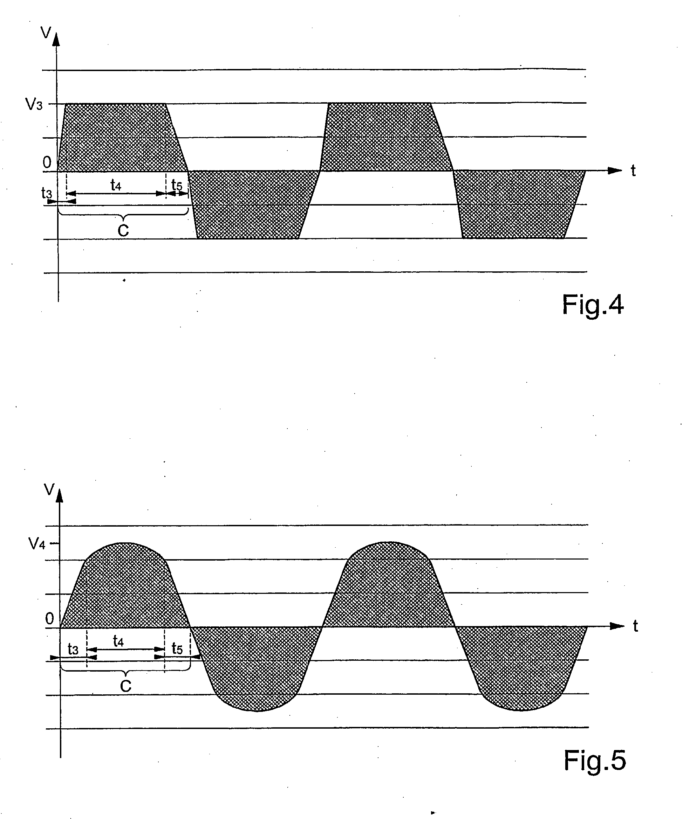

La figure 4 est une représentation graphique d'une autre forme de tension appliquée aux bornes du moteur de l'instrument selon le procédé de commande de l'invention. Comme on le constate immédiatement à l'examen de la figure, la tension appliquée aux bornes du moteur présente un profil trapézoïdal plutôt que triangulaire. Plus précisément, le moteur est accéléré pendant une durée t₃ de l'ordre de 57 ms pour le cas où le moteur oscille à une fréquence de 3 Hz et où la lame coupante effectue six tours complets entre deux changements de sens successifs. Le moteur est ensuite maintenu à vitesse constante durant un laps de temps t₄ de l'ordre de 83 ms puis est décéléré et arrêté en prévision de l'inversion de son sens de marche sur une durée d'environ 30 ms, les consignes de fonctionnement de la lame coupante étant celles indiquées ci-avant. La tension V₃ est inférieure aux tensions V₁, V'₁.

Comme on l'aura compris de ce qui précède, on dispose de moins de temps que dans l'art antérieur pour accélérer le moteur. Par conséquent, la vitesse maximale atteinte par le moteur lorsqu'il est commandé selon le procédé de l'invention est inférieure à ce qu'elle était auparavant. Avec des consignes de fonctionnement de la lame coupante qui sont celles indiquées ci-dessus, le moteur tourne à une vitesse maximale de l'ordre de 27'000 à 30'000 tours/minute. Le moteur tourne donc approximativement 33% moins vite en comparaison avec l'art antérieur, mais comme il respecte un palier à cette vitesse de l'ordre de 83 ms, les consignes de fonctionnement de la lame coupante sont malgré tout respectées.

L'avantage immédiat qui ressort du procédé de l'invention est que, la vitesse maximale atteinte par le moteur étant moindre, les à-coups liés à l'inertie du rotor du moteur sont atténués lorsqu'on ralentit ledit moteur. L'instrument est donc d'une utilisation beaucoup plus confortable et moins fatigante pour le praticien. En outre, le geste opératoire gagne en précision, ce qui permet d'éviter au patient des douleurs inutiles.

La Demanderesse est également parvenue à démontrer que, malgré la diminution de la vitesse maximale du moteur, on conserve la même qualité de découpe des tissus. Ce faisant, la Demanderesse va à l'encontre d'un préjugé bien établi dans l'état de la technique selon lequel plus la vitesse de rotation du moteur est élevée, meilleure est la qualité de découpe des tissus.

On notera également que la durée réservée au moteur pour atteindre sa vitesse de palier est plus courte qu'auparavant. Par conséquent, durant le bref intervalle de temps où l'on accélère le moteur, le pic de courant est plus élevé qu'il ne l'était par le passé, sans toutefois que la Demanderesse ait pu constater d'effet indésirable sur le moteur.

Enfin, vu la vitesse maximale relativement basse à laquelle tourne le moteur selon le procédé de l'invention, on dispose d'une souplesse remarquable pour adapter le fonctionnement du moteur en fonction des consignes de fonctionnement de la lame coupante. Au contraire, si l'on souhaite augmenter la tension V'₁ appliquée aux bornes du moteur pour le faire tourner à plus grande vitesse encore que dans le cas illustré à la figure 2A, on risque alors de dépasser les 50 Volts aux bornes du moteur. Or, dans le domaine de l'instrumentation électrique médicale, il s'agit d'une valeur de tension qui, pour des raisons bien évidentes de sécurité, ne doit habituellement pas être dépassée. Par conséquent, en cherchant à éviter les à-coups trop brusques avec les procédés de commande de l'art antérieur, les performances du moteur se dégradent et l'on jouit d'une très faible marge de manoeuvre en termes d'adaptation de la vitesse dudit moteur en fonction des consignes de fonctionnement de la lame coupante.

Il va de soi que la présente invention n'est pas limitée au mode de fonctionnement du moteur selon l'invention, et que diverses modifications et variantes simples peuvent être envisagées par l'homme du métier sans sortir du cadre de l'invention tel que défini par les revendications annexées. En particulier, il peut également être envisagé d'appliquer aux bornes du moteur un signal de commande de forme pseudo-sinusoïdale comme illustré à la figure 5. On voit en effet sur cette figure que, durant une fraction de la partie centrale t₄ du cycle C, le signal de commande appliqué au moteur continue de croître mais avec une pente moins accentuée que durant la partie initiale t₃ de ce même cycle C. Autrement dit, on continue d'accélérer le moteur mais moins rapidement qu'au cours de la phase initiale t₃. Puis, durant une seconde fraction de la partie centrale t₄ du cycle C, on commence à décélérer le moteur, mais moins rapidement que durant la partie finale t5 du cycle C. Comme on le comprendra aisément, une telle forme du signal de commande répond elle aussi à la définition générale du procédé selon l'invention. En effet, durant la partie centrale t₄ du cycle C, la pente du signal de commande appliqué au moteur est, en valeur absolue, inférieure à la pente, toujours en valeur absolue, de ce même signal durant les phases initiale t₃ et finale t₅. La tension V₄ est inférieure aux tensions V₁, V'₁. En résumé, le but de la présente invention est de réduire la vitesse du moteur pour travailler en palier à une vitesse environ 30% plus basse que la vitesse habituellement observée avec les dispositifs de l'art antérieur, ce qui permet de réduire sensiblement les à-coups dans le moteur tout en conservant une qualité de découpe des tissus et des performances de la lame (nombre de tours, fréquence d'oscillation) inchangées.

## Revendications

1. Procédé de commande bidirectionnelle d'un moteur électrique qui entraîne alternativement dans un sens et dans le sens opposé une lame coupante d'un instrument chirurgical ou dentaire, **caractérisé en ce qu'**il consiste à appliquer au moteur, au cours d'un cycle (C) entre deux inversions successives du sens d'entraînement de la lame coupante, un signal de commande qui présente un profil dont la pente, en valeur absolue, est essentiellement inférieure durant une partie centrale (t₄) du cycle (C) à la pente, toujours en valeur absolue, d'une partie initiale (t₃) dudit cycle (C) au cours de laquelle on accélère le moteur et d'une partie finale (t₅) du cycle (C) au cours de laquelle on décélère le moteur.

2. Procédé selon la revendication 1, **caractérisé en ce que** la forme du signal de commande appliqué au moteur est sensiblement carrée ou rectangulaire ou bien encore trapézoïdale.

3. Procédé selon la revendication 2, **caractérisé en ce qu'**entre deux inversions successives du sens d'entraînement de la lame coupante, on commence par accélérer le moteur pendant la durée (t₃), puis on le maintient à vitesse constante pendant une durée (t₄) avant de le décélérer et de l'arrêter en prévision de sa prochaine inversion de sens pendant une durée (t₅).

4. Procédé selon l'une quelconque des revendications 1 à 3, **caractérisé en ce que**, au plus, on fait tourner le moteur à une vitesse inférieure d'environ 30% à la vitesse maximale qu'il peut atteindre.

5. Procédé selon l'une quelconque des revendications 1 à 4, **caractérisé en ce que**, durant la partie centrale du cycle, le moteur tourne à une vitesse de l'ordre de 27'000 à 30'000 tours/minute.

6. Procédé selon l'une quelconque des revendications 2 à 5, **caractérisé en ce que**, si la fréquence d'oscillation de la lame coupante est de 3 Hz et si cette lame effectue six tours dans chaque sens, on accélère le moteur pendant environ 57 ms, on le maintient à vitesse constante pendant environ 83 ms et on le décélère pendant environ 30 ms avant de l'arrêter et d'inverser son sens de rotation.

## Patentansprüche

1. Verfahren zur bidirektionalen Steuerung eines Elektromotors, welche ein Schneidmesser eines chirurgischen oder zahnärztlichen Instruments in einer Richtung und in einer Gegenrichtung alternierend antreibt, **dadurch gekennzeichnet, dass** es darin besteht während eines Zyklus (C) zwischen zwei aufeinanderfolgenden Umkehrungen der Antriebsrichtung des Schneidmessers den Motor mit einem Steuersignal zu beaufschlagen, welches ein Profil aufweist, dessen Steigung, im Absolutwert, in einem zentralen Abschnitt (t₄) des Zyklus (C) wesentlich geringer ist als die Steigung, immer im Absolutwert, eines Anfangsabschnitts (t₃) des Zyklus (C), während welchem der Motor beschleunigt, und eines letzten Abschnitts (t₅) des Zyklus (C) während welchem der Motor verzögert.

2. Verfahren nach Anspruch 1, **dadurch gekennzeichnet, dass** die Form des den Motor beaufschlagenden Steuersignals in etwa quadratisch, rechteckig oder trapezförmig ist.

3. Verfahren nach Anspruch 2, **dadurch gekennzeichnet, dass** zwischen zwei aufeinanderfolgenden Umkehrungen der Antriebsrichtung des Schneidmessers man den Motor während der Zeitdauer (t₃) beschleunigt, dann bei einer konstanten Geschwindigkeit während einer Zeitdauer (t₄) belässt, bevor man ihn im Vorgriff auf seine nächste Umkehrung der Richtung während einer Zeitdauer (t₅) verzögert und anhält.

4. Verfahren nach einem der Ansprüche 1 bis 3, **dadurch gekennzeichnet, dass** man den Motor zumindest bei einer Geschwindigkeit betreibt, die in etwa 30 % geringer als die Maximalgeschwindigkeit ist, die er erreichen kann.

5. Verfahren nach einem der Ansprüche 1 bis 4, **dadurch gekennzeichnet, dass** der Motor während des zentralen Abschnitts des Zyklus bei einer Geschwindigkeit von etwa 27.000 bis 30.000 Umdrehungen / Minute läuft.

6. Verfahren nach einem der Ansprüche 2 bis 5, **dadurch gekennzeichnet, dass** wenn die Schwingungsfrequenz des Schneidmessers 3 Hz ist und wenn das besagte Schneidmesser sechs Umkehrungen in jede Richtung vollzogen, beschleunigt man den Motor während etwa 57 ms, belässt man den Motor bei einer konstanten Geschwindigkeit über etwa 83 ms, und verzögert man den Motor während 30 ms vor einem Anhalten und vor einem Umkehren seiner Drehrichtung.

## Claims

1. Method for the bidirectional control of an electric motor which alternately drives a cutting blade of a surgical or dental instrument in one direction and in the opposite direction, **characterized in that** the method consists in applying to the motor, during a cycle (C) between two successive reversals of the drive direction of the cutting blade, a control signal which has a profile wherein the slope, in absolute value, is essentially smaller during a central part (t4) of the cycle (C) than the slope, also in absolute value, of an initial part (t3) of said cycle (C) during which the motor is accelerated and of a final part (t5) of the cycle (C) during which the motor is decelerated.

2. Method according to claim 1, **characterized in that** the shape of the control signal applied to the motor is substantially square or rectangular or even trapezoidal.

3. Method according to claim 2, **characterized in that**, between two successive reversals of the drive direction of the cutting blade, the motor is first accelerated during the duration (t3), then the motor is maintained at constant speed during a duration (t4) before being decelerated and stopped prior to the next reversal of direction during a duration (t5).

4. Method according to any of claims 1 to 3, **characterized in that**, at most, the motor is rotated at a speed approximately 30% lower than the maximum speed the motor can reach.

5. Method according to any of claims 1 to 4, **characterized in that**, during the central part of the cycle, the motor rotates at a speed on the order of 27,000 to 30,000 revolutions per minute.

6. Method according to any of claims 2 to 5, **characterized in that**, if the oscillation frequency of the cutting blade is 3Hz and if the blade makes six revolutions in each direction, the motor is accelerated for approximately 57ms, maintained at constant speed for approximately 83ms and decelerated for approximately 30ms before the motor is stopped and the direction of rotation is reversed.
